(19) **Europäisches Patentamt / European Patent Office / Office européen des brevets**

(11) **EP 2 404 980 A1**

(12) **EUROPEAN PATENT APPLICATION**

(43) Date of publication:
**11.01.2012 Bulletin 2012/02**

(21) Application number: **10305759.2**

(22) Date of filing: **08.07.2010**

(51) Int Cl.:
*C10G 25/05* (2006.01)　　*C10G 29/20* (2006.01)
*C10G 45/32* (2006.01)　　*C10G 50/00* (2006.01)
*C10G 69/12* (2006.01)　　*C07C 2/00* (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Total Raffinage Marketing**
**92800 Puteaux (FR)**

(72) Inventors:
• **Minoux, Delphine**
　**B-1400, NIVELLES (BE)**
• **Revault, Cyril**
　**76310, SAINTE-ADRESSE (FR)**

(74) Representative: **Largeau, Béatrice et al**
**Cabinet Jolly**
**54, rue de Clichy**
**75009 Paris (FR)**

(54) **Hydrocarbon feedstock average molecular weight increase**

(57)　The invention deals with hydrocarbon feedstock molecular weight increase via olefin oligomerization and/or olefin alkylation onto aromatic rings.

Addition of a purification section allows improved unit working time and lower maintenance.

EP 2 404 980 A1

**Description**

Hydrocarbon feedstock average molecular weight increase. The instant invention discloses a method for the conversion of low boiling point olefin containing hydrocarbon feedstock into higher boiling point cuts via olefin oligomerization and/or olefin alkylation onto aromatic moieties.

More specifically, the processed hydrocarbon load has an initial boiling point that is set between butanes to hexanes boiling points (included) and a final boiling point equal to or below 165°C.

Refineries of today have to adapt to a continuously evolving and fluctuating market, requiring always more flexibility. It is especially the case with the gasoline/middle distillates markets, which have largely evolved during the years: a shift in product focus from gasoline to middle distillates is being observed in the current and future European market demands.

To respond to the above-mentioned disequilibrium, a nice way of readjusting the gasoline/diesel balance according to the market needs consists in upgrading at least part of the gasoline into middle distillates (jet, diesel).

In a typical refinery today, most of the C4-C8 molecules end up in the gasoline-pool. It is important to note that only around 5% of these molecules were initially present in the crude oil as delivered, while cracking during refinery processing creates the rest. About 50% of the C4's and 40% of the C5's that are produced during Fluidized Catalytic Cracking (FCC) are olefinic in nature. Currently the C4 olefins are used as feed for the alkylation and etherification units to create gasoline components with high octane and the higher olefins are generally directly blended into the gasoline pool.

In that context, a convenient solution that allows a renewed equilibrium between gasoline and distillates would be to convert unsaturated molecules (olefins and/or aromatics) contained in the gasoline feed into heavier molecules lying in the middle distillate range (i.e. diesel and kerosene) by selective oligomerization and/or alkylation of these unsaturated molecules.

The present invention relates to a process for the manufacture of higher molecular weight organic molecules from a stream of lower molecular weight molecules which contain contaminants brought in by the feedstock.

Oligomerization of olefinic streams is largely documented and is a widely used commercial process, but is subject to limitations.

Typically, oligomerization processes involve contacting lower olefins (typically mixtures of propylene and butenes) coming from Fluid Catalytic Cracker (FCC) and/or steam crackers with a solid acid catalyst, such as Solid Phosphoric acid (SPA) catalyst, crystalline molecular sieve, acidic ion exchange resin or amorphous acid material (silico-alumina).

With SPA catalyst, the pressure drop over the catalytic bed(s) increases gradually due to coking, swelling of the catalyst, and is therefore the limiting factor of a run duration, the unit being shutdown once the maximum allowable pressure drop has been reached.

With crystalline molecular sieve, acidic ion exchange resin or amorphous acidic material (silico-alumina), the limiting factor is usually no more the pressure drop along the catalytic bed but the reactor run length which is determined by the catalytic performances (shutdown when the catalytic activity has dropped to an unacceptably low level). The performances of such catalyst are therefore sensitive to poisons contained in the feedstock, which may considerably affect the cycle length.

Certain impurities such as sulfur containing contaminants and basic nitrogen have an adverse effect in the useful lifetime of the catalyst.

Among the sulfur containing contaminants, low molecular weight sulfur species are especially troublesome, as described in US 2008/0039669, i.e. aliphatic thiols, sulfides and disulfides. For example dimethyl-, diethyl-, and ethyl-methyl-sulphides, n-propane thiol, 1-butane thiol and 1,1-methylethyl thiol, ethyl-methyl- and dimethyl- disulphides, and tetrahydrothiophene.

Among the basic nitrogen contaminants, we can distinguish:

- The strong organic Bronsted bases (characterized by at least one hydrogen atom bound to the nitrogen atom, and being proton acceptors), such as amines and amides, contribute to negatively affect the catalyst performances.
- The other organic nitrogen compounds, called Lewis bases, have free electron pair on the nitrogen atom such as nitriles, morpholines or N-Methyl pyrrolidone. Though much weaker bases as compared to the Bronsted bases, they strongly deactivate the catalyst. The detrimental effect of such impurities has been discussed in US patent application publication 2008/0312484.

In some specific cases, the purity of the olefinic stream is not an issue:

It is the case when the stream involves very pure Fisher-Tropsch (FT) derived olefins (US2008/0257783 or WO2006/091986)

It is also the case in the fully integrated system MTG (Methanol-to-Gasoline) where olefinic streams are produced from the Methanol-To-Olefin process and oligomerized through the so-called MOGD process (Mobil Olefin to Gasoline and Distillate). The MOGD process, proposed by Mobil (US-4,150,062; US-4,227,992; US-4482772; US-4506106; US-4543435) and developed between the seventies' and eighties', in fact used ZSM-5 zeolite as catalyst. The products obtained from the reaction of butenes are trimers and tetramers, characterized by a low branching degree. The gas oil fraction however is lower than that of the jet fuel fraction and consequently, even if this process offers good quality gas oil (cetane number > 50), it is more interesting for the production of jet fuel than gas oil. In US20040254413 patent application, ExxonMobil pursued the Mobil development and introduced the new generation of MOGD. This invention uses two or more zeolite catalysts. Examples of zeolite catalysts include a first catalyst containing ZSM-5, and a second catalyst containing a 10-ring molecular sieve, including but not limited to, ZSM-22, ZSM-23, ZSM35, ZSM-48, and mixtures thereof. The ZSM-5 can be unmodified, phosphorous modified, steam modified having a micropore volume reduced to not less than 50% of that of the unsteamed ZSM-5, or various mixtures thereof.

ZSM-5 stands for Zeolite Sieve of Molecular porosity (or Zeolite Socony Mobil) - 5, (structure type MFI-Mordenite Framework Inverted). ZSM-5 is an aluminosilicate zeolite mineral belonging to the pentasil family of zeolites. Its chemical formula is $Na_nAl_nSi_{96-n}O_{192} \cdot 16H_2O$ (0<n<27).

In a similar manner, Lurgi AG, Germany (WO2006/076942), has developed the Methanol to Synfuels (MTS) process, which is in principle similar to the MOGD process. The Lurgi route is a combination of simplified Lurgi MTP technology with COD technology from Sued Chemie (US5063187). This process produces gasoline (RON 80) and diesel (Cetane ~ 55) in the ratio of approximately 1:4. Disclosed is a method for the production of synthetic fuels, wherein, in a first step, a gas mixture consisting of methanol and/or dimethyl ether and/or another oxygenate and water vapor is reacted at temperatures of 300-600 °C in order to form olefins with, preferably, 2-8 carbon atoms. In a second step, the olefin mixture thus obtained is oligomerized at an elevated pressure to form higher olefins with predominantly more than 5, preferably 10-20 carbon atoms. According to said method, a) the production of olefins in the first step is carried out in the presence of a gas flow which essentially consists of saturated hydrocarbons which are separated from the product flow of the second step and returned to the first step, and (b) the production of olefins is carried out in the second step in the presence of a flow of water vapor which is separated from the product flow of the first step and returned to the first step.

Above-discussed methods are hardly developable in the context of gasoline upgrading into distillates: Commonly available olefinic feedstocks cause rapid deactivation of existing oligomerization catalysts, due to the presence of contaminants in the feed, which is a critical issue.

Catalytic cracking, usually fluid catalytic cracking (FCC), is a suitable source of cracked naphthas. Thermal cracking processes such as coking may also be used to produce usable feeds such as coker naphtha, pyrolysis gasoline, and other thermally cracked naphthas.

The process may be operated with a part of, or the entire gasoline fraction, obtained from a catalytic or thermal cracking step.

In case of alkylation reactions, the co-feed comprises a light fraction, boiling within the gasoline boiling range which is relatively rich in aromatics. A suitable refinery source for the light fraction is a reformate fraction. The reformate co-feeds usually contain very low amounts of sulfur as they have usually been subjected to desulfurization prior to reforming.

To cope with the contaminants issue, different techniques have been proposed:

A first technique consists in contacting the nitrogen and sulfur contaminated feedstock either with a hydrotreating catalyst at oxidized state (US 6,884,916 - Exxon) or with a metal oxide catalyst (US7,253,330) in the absence of hydrogen, ahead of the oligomerization section, thus limiting catalyst deactivation. The pretreatment is believed to convert small sulfur compounds into larger sulfur species, then into more sterically hindered molecules, no more entering the catalyst pores, and limiting catalyst deactivation.

Another convenient way (US 7,186,874 - Exxon) is to mitigate the adverse effect of the sulfur compounds on catalyst activity by appropriately adjusting the operating conditions of the process by, for example, temperature rising.

Removal of nitriles and other organic nitrogen-containing Lewis bases from the oligomerization feed may be achieved by a washing step with water (WO2007/104385 - Exxon). Removal of basic nitrogen and sulfur-containing organic compounds by scrubbing with contaminant removal washes such as caustic, methyl-ethyl-amine (MEA), or other amines or aqueous washing liquids, is discussed in WO 2006/094010 (Exxon). This method allows contaminants to stand at acceptable levels (10-20 ppmwt S, trace levels for N) and therefore to limit catalyst deactivation prior to oligomerization and alkylation reactions.

Sorption techniques are also reported for nitrogen components removal from the feed. US2005/0137442 (UOP) discloses the use of molecular sieves catalysts (such as Y-zeolite) to remove the nitrogen-based contaminants present in an olefinic stream to be alkylated. Specificity of US2005/0137442 (UOP) lies in operating conditions: adsorption is conducted at a temperature of at least 120°C to increase the nitriles adsorption capacity of the sorbent in the presence of water.

Purification section using molecular sieves is also reported in EP1433835 (IFP), where shaped MOR catalyst having a Si/Al atomic ratio of 45 allows decreasing nitrogen content from 10ppmwt to 0.2ppmwt. US2008/0312484 (Exxon) shown that such a low nitrogen concentration can be tolerated in olefin-containing hydrocarbon feeds loaded in oligomerization sections.

WO2006/067305 (IFP) discloses a process for producing propylene from C4/C5 cut (from steam cracking or catalytic cracking). Prior to the steps of so-called "oligomerization/cracking", the following purification sequence is used to remove contaminants: a selective hydrogenation is used to convert the dienes and acetylenic compounds into mono-olefins, then drying and desulfurization steps are performed by the use of different sorbents (3A, 13X molecular sieves).

Thus, state of the art processes do not use untreated refinery streams for oligomerization/ alkylation but rather pure streams (such as ex-FT or ex-MTO olefins). As such, existing commercial solutions cannot give satisfactory results for untreated refinery streams to be valorized.

It has now been found an improved route to process untreated refinery streams (such as FCC, coker ...) into an oligomerization/alkylation reaction.

A first object of the invention concerns a method for the treatment of an olefin containing hydrocarbon feedstock, wherein said feedstock successively undergoes (i) selective hydrogenation, (ii) treatment on adsorbent to obtain at least one nitrogen depleted fraction, (iv) at least one middle distillate generation via (iv-a) oligomerization of said nitrogen depleted fraction and/or (iv-b) alkylation of said nitrogen depleted fraction.

A first step consists in selective hydrogenation of di-olefins into mono-olefins to avoid gum formation in downstream catalyst, allowing at the same time conversion of low molecular weight sulfur containing molecules (aliphatic thiols, sulfides or disulfides being especially troublesome) into heavier molecular weight sulfur containing molecules.

Preferably, the resulting stream is then fractionated in a splitter in a light cut (LCCS gasoline), and a heavier cut (HCCS or mixed MCCS/HCCS)

A further step consists in removing from the obtained LCCS gasoline cut (or from unfractionated selectively hydrogenated feedstock) the residual light N and S compounds by the use of different sorbents (alone or in combination, such as 3A/13X molecular sieves) before entering the oligomerization/alkylation section.

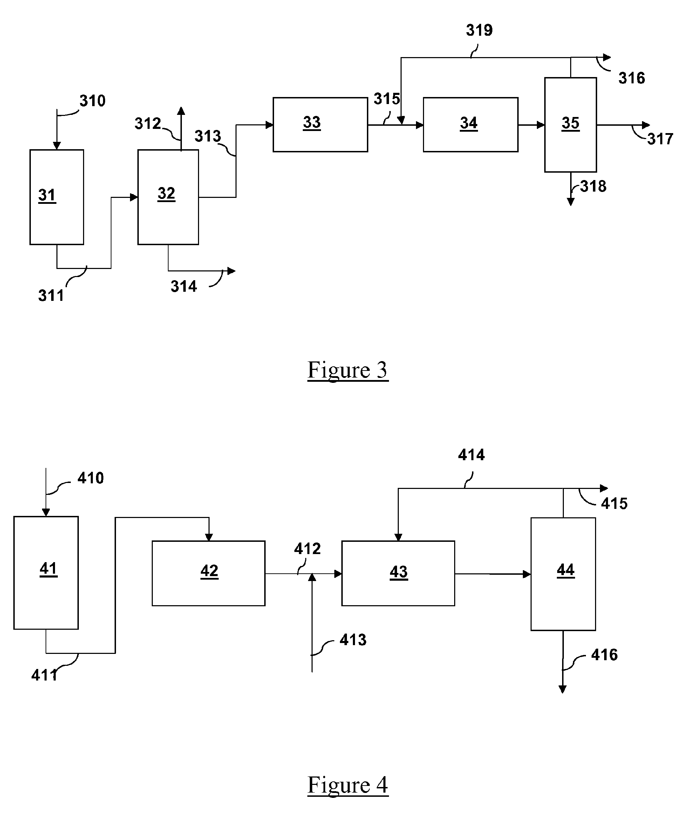

Once purified in such a purification section, the olefinic stream can then be valorized into middle distillates by oligomerization and/or alkylation as represented on figures 3 to 8.

It has been found that oligomerization and/or alkylation catalyst lifetime is tremendously increased using this method. An hypothesis to explain improvement in catalyst lifetime in operation could be that the allegedly most detrimental sulfur containing compounds (S compounds) (such as aliphatic thiols, sulfides and disulfides: for example, dimethyl-, diethyl-, and ethyl-methyl-sulphides, n-propane thiol, 1-butane thiol and 1,1-methylethyl thiol, ethylmethyl- and dimethyl-disulphides) are converted within the selective hydrogenation unit by direct reaction of light sulfur containing molecules (e.g. here above-mentioned S compounds) with olefins or thiophenic compounds, thus leading to the formation of heavier S molecules, no more present in the LCCS cut issued from the splitter section.

Though thiophenic ring containing molecules are still present in the LCCS cut, they do not interact significantly with the acid sites of the oligomerization/alkylation catalyst.

The allegedly most detrimental nitrogen containing compounds (N molecules), if not converted into heavier N molecules in a similar manner as S molecules, are removed in the purification section by adsorption on sorbents. It is advantageous to use molecular sieves (3A, 13X, HY ...), Acidic ion-exchange resins, Acid-treated clays, Activated aluminas such as SAS-351, MOF (Metal Organic Frameworks), amorphous alumina-silica (ASA), Spent FCC catalysts either alone or in combination.

Advantageously, the method of the invention comprises a further step wherein (v) at least one unreacted material is separated from said at least one middle distillate.

Advantageously, said at least one unreacted material is recycled at step (iv) for at least one middle distillate generation.

In the method of the invention, selectively hydrogenated feedstock may be splitted into at least two of LCCS (Light Catalytic Cracked Stream), MCCS (Middle Catalytic Cracked Stream), HCCS (Heavy Catalytic Cracked Stream) and fuel gas prior to treatment of at least one of the foregoing on adsorbent.

The alkylation of said at least one nitrogen depleted fraction is advantageously performed in presence of an aromatic containing hydrocarbon feedstock.

In a first embodiment of the above described method, said feedstock successively undergoes (i) selective hydrogenation, (ii) splitting into a light cut (LCCS) and a heavier cut (HCCS or mixed MCCS/HCCS), (iii) LCCS treatment on adsorbent to obtain a nitrogen depleted LCCS, (iv) a first middle distillate generation via (iv-a) oligomerization of said nitrogen depleted LCCS and/or (iv-b) alkylation of said nitrogen depleted LCCS.

Preferably, alkylation is performed in presence of an aromatic containing stream.

Advantageously, the method of the invention comprises a further step (v) wherein a first unreacted material is separated from said first middle distillate.

Advantageously, said first unreacted material is recycled at step (iv) for first middle distillate generation.

The method may also comprise another step (vi) wherein said first middle distillate is further alkylated with a gasoline cut to produce a second middle distillate.

Preferably, (vii) a second unreacted material is then separated from said second middle distillate and more particularly, said second unreacted material is recycled at step (vii) for said second middle distillate generation.

In a second embodiment of the above described method, said feedstock successively undergoes (i) selective hydrogenation, (ii) splitting into a light cut (LCCS) and a heavier cut (HCCS or mixed MCCS/HCCS), (iii) said LCCS and said HCCS or mixed MCCS/HCCS separate treatment on adsorbent so as to remove nitrogen compounds, to obtain nitrogen depleted LCCS and nitrogen depleted HCCS or mixed MCCS/HCCS, (iv) a third middle distillate generation via (iv-a) oligomerization and/or (iv-b) alkylation of said nitrogen depleted LCCS combined with said nitrogen depleted HCCS or mixed MCCS/HCCS.

A third unreacted material may be separated from said third middle distillate. A gasoline cut may also be separated from said third middle distillate. Preferably, said third unreacted material is recycled at step (iv) for said third middle distillate generation.

In a third embodiment of the invention, said feedstock successively undergoes (i) selective hydrogenation, (ii) splitting into a light cut (LCCS) and a heavier cut (HCCS or mixed MCCS/HCCS), (iii) said LCCS and said HCCS or mixed MCCS/HCCS separate treatment on adsorbent so as to remove nitrogen compounds, to obtain nitrogen depleted LCCS and nitrogen depleted HCCS or mixed MCCS/HCCS, (iv-a) a fourth middle distillate generation via oligomerization of said nitrogen depleted LCCS, (iv-b) a fifth middle distillate generation via alkylation of said nitrogen depleted HCCS or mixed MCCS/HCCS.

Preferably, alkylation is performed in presence of an aromatic containing stream.

Unreacted olefins are advantageously separated from said fourth middle distillate. For example, said unreacted olefins are recycled at step (iv-a) for fourth middle distillate generation.

Fourth middle distillate may also be alkylated with nitrogen depleted HCCS or mixed MCCS/HCCS and said aromatic containing stream at step (iv-b) to produce said fifth middle distillate. Advantageously, unreacted material is then separated from said fifth middle distillate, and preferably, said unreacted material is recycled at step (iv-b).

In any of the embodiments of the invention, said adsorbent comprises one or more of molecular sieves, acidic ion-exchange resins, acid-treated clays, activated aluminas, spent FCC catalysts, MOF (Metal-Organic Framework), ASA, NiMo, and catalysts guard beds.

Preferably, the adsorbent is selected among, or is a combination of one or more of 13X molecular sieve, ASA, NiMo, and MOF.

Advantageously, adsorbent used in any method of the invention is loaded into a purification section located in a guard bed capacity.

With regards to purification section, a guard bed reactor may be operated on a swing cycle with two beds, one bed being used on stream for contaminant removal and the other on regeneration in the conventional manner. If desired, a three-bed guard bed system may be used on a swing cycle with the two beds used in series for contaminants removal and the third bed in regeneration. With the three-bed guard bed system used to achieve low contaminant levels by the two-stage series sorption, the beds will pass sequentially through a three-step cycle of regeneration. A three-bed guard bed system allows better use of guard bed sorption capacity since non-used sorbent sent in regeneration is lowered, if not eliminated. Typically, a three-bed guard bed system may be operated as follows: Step 1: feedstock flows into first then second guard bed, third one being isolated and under regeneration. Step 2: once first guard bed is saturated in impurities, the latter is isolated and regenerated, and feedstock now flows into second then third guard beds. Step 3: once second guard bed is saturated in impurities, the latter is isolated and regenerated, and feedstock now flows into third then first guard beds. Step 4: go to step 1.

A plural reactor system may be employed with inter-reactor cooling for oligomerization/alkylation, whereby exothermal reaction can be carefully controlled to prevent excessive temperature above the normal moderate range.

The oligomerization/alkylation reactor can be of isothermal or adiabatic fixed bed type or a series of such reactors or a moving bed reactor. The oligomerization/alkylation may be performed continuously in a fixed bed reactor configuration using a series of parallel "swing" reactors. Herein used catalysts have been found to be stable enough. This enables the oligomerization/alkylation process to be performed continuously in two parallel "swing" reactors wherein, when one or two reactors are in operation, the other reactor is undergoing catalyst regeneration. Catalysts of the invention may be regenerated. Regeneration may be done several times.

An object of the present invention is to convert olefins containing stream into heavier hydrocarbons enriched distillate, employing a continuous multi-stage catalytic technique. A plural reactor system may be employed with inter-reactor cooling, whereby the exothermal reaction can be carefully controlled to prevent excessive temperature above the normal moderate range. Preferably, the maximum temperature differential across only one reactor does not exceed 75° C. Optionally, the pressure differential between the two stages can be utilized in an intermediate flashing separation step.

The following types of acid catalysts can be used in oligomerization and/or alkylation:

Amorphous or crystalline alumosilicate or silicaalumophosphate in H-form, optionally containing alkali, alkali-earth, transition or rare-earth elements, selected from the group:

MFI (e.g. ZSM-5, silicalite-1, boralite C, TS-1), MEL (Si/Al >25) (e.g. ZSM-11, silicalite-2, boralite D, TS-2, SSZ-46), ASA (amorphous silica-alumina), MSA (mesoporous silica-alumina), FER (e.g. Ferrierite, FU-9, ZSM-35), MTT (e.g. ZSM-23), MWW (e.g. MCM-22, PSH-3, ITQ-1, MCM-49), TON (e.g. ZSM-22, Theta-1, NU-10), EUO (e.g. ZSM-50, EU-1), ZSM-48, MFS (e.g. ZSM-57), MTW, MAZ, SAPO-11, SAPO-5, FAU (e.g. USY), LTL, BETA, MOR, SAPO-40, SAPO-37, SAPO-41, MCM-41, MCM-48 and a family of microporous materials consisting of silicon, aluminum, oxygen and optionally boron, $Al_2O_3$, and mixtures thereof.

Amorphous alumosilicates or silicaalumophosphates in H-form optionally modified by addition of halogens (Fluorine preferred) such as MSA (mesoporous silica-alumina) can also be used.

Above-mentioned catalysts can be subjected to an additional treatment before use, including ion exchange, modification with metals such as alkali, alkali-earth and rare earth metals, steaming, treatment in an alkaline medium, acid treatment or other dealumination methods, phosphatation, surface passivation by silica deposition or combination thereof.

The amount of alkali, alkali-earth, transition or rare-earth elements is in the range 0.05-10wt%, preferably from 0.1 to 5wt%, more preferably from 0.2 to 3wt% (wt% stands for weight percent).

Preferred alkali, alkali-earth or rare-earth elements are selected among Na, K, Mg, Ca, Ba, Sr, La, Ce, and mixtures thereof.

Above-mentioned catalysts may be additionally doped with further metals. In this respect, and according to another embodiment of the invention, Me-catalysts (Me = metal) containing at least 0. 1wt% are used. Preferably, the metal is selected from the group of Zn, Mn, Co, Ni, Ga, Fe, Ti, Zr, Ge, Sn, Cr, and mixtures thereof.

Those atoms can be inserted into the tetrahedral framework through a $[MeO_2]$ tetrahedral unit. Incorporation of the metal component is typically accomplished during synthesis of the molecular sieve. However, post-synthesis ion exchange or impregnation can also be used. In post-synthesis exchange, the metal component will be introduced as a cation on ion-exchange positions at an open surface of the molecular sieve, but not into the framework itself.

The selected materials could be subjected to a different treatment before use in the reaction, including introduction of phosphorous, ion exchange, modification with alkali, alkali-earth or rare earth metals, steaming, acid treatment or other dealumination methods, surface passivation by silica deposition or combination thereof.

The catalyst can be a blend of materials as depicted above, and/or can be further combined with other materials that provide additional hardness or catalytic activity to the finished catalyst product (binder, matrix).

The method of the invention permits to treat a feedstock issued from FCC, coker, flexi-coker, visbreaker, steam cracker, hydrocracker, for example from DHC (distillate hydrocracker) or MHC (mild hydrocracker) hydrocracker, preferably from FCC or coker.

The final boiling point of the feedstock may be below 200°C, preferably below 165°C.

The initial boiling point of the feedstock may be above -50°C, preferably above 0°C, more preferably above +25°C.

The invention is now described with reference to appended figures 1-8, which depict different non-limitative methods for the preparation of middle distillate cuts starting from olefin containing lighter cuts, e.g. ex-FCC, ex-coker or ex-DHC gasoline cuts.

Figure 1 is a graph showing olefin conversion (%wt) as a function of TOS (Time Of Stream) when no purification section is used. (See example 1 for details)

Figure 2 shows olefin conversion rate (%wt) as a function of TOS when a purification section is used. (See example 2 for details)

Figure 3 represents an oligomerization method using a purification section and optionally a recycle of unreacted stream.

Figure 4 shows an oligomerization and alkylation process scheme using a purification section and optionally a recycle of unreacted stream.

Figure 5 illustrates another alkylation method using a purification section and optionally a recycle of unreacted olefin and aromatic streams.

Figure 6 represents another oligomerization and alkylation process using a purification section and optionally a recycle of unreacted olefin and aromatic streams.

Figure 7 shows a one-pot oligomerization and alkylation scheme using a purification section and optionally a recycle of unreacted olefin stream.

Figure 8 represents an oligomerization and alkylation scheme of full olefin containing refinery stream using a purification section and optionally a recycle of unreacted olefin and aromatic streams.

Figure 3 represents an oligomerization method using a purification section and optionally a recycle of unreacted stream.

The untreated refinery stream 310 is fed to a Selective Hydrogenation Unit (SHU) 31. The dedienized refinery stream 311 thus obtained is fed to a splitter 32 wherein it is separated in fuel gas 312, LCCS 313 (FBP = 60°C to 100°C) and HCCS 314 (Boiling point cut ranges from 60-170°C to 100-170°C).

In the present application, FBP stands for Final Boiling Point and IBP stands for Initial Boiling Point.

LCCS 313 is then fed to a purification unit 33 for removal of nitrogen by adsorption on sorbents.

The purified LCCS stream 315 obtained is fed to an oligomerization unit 34, the obtained products being separated in a further splitter 35 into a gasoline 316, aromatics 317 and middle distillate 318.

The part of the gasoline 316, consisting of unreacted olefins, may optionally be recycled back into the oligomerization unit 34 via the line 319.

Figure 4 shows an oligomerization and alkylation process scheme using a purification section and optionally a recycle of unreacted stream.

The untreated refinery stream 410 (for example LCN (Light Cracked Naphtha)) is fed to a Selective Hydrogenation Unit, SHU 41. The dedienized refinery stream 411 thus obtained is purified in a purification units 42 for removal of nitrogen by adsorption on sorbents.

The purified stream 412 is fed to an oligomerization unit 43. Optionally, an aromatic stream 413 may be co-fed with stream 412.

The effluents issued from the oligomerization unit 43 are separated in a splitter 44 into gasoline 415 and middle distillates 416.

The part of the gasoline 415, consisting of unreacted material, may optionally be recycled back into the oligomerization unit 43 via the line 414.

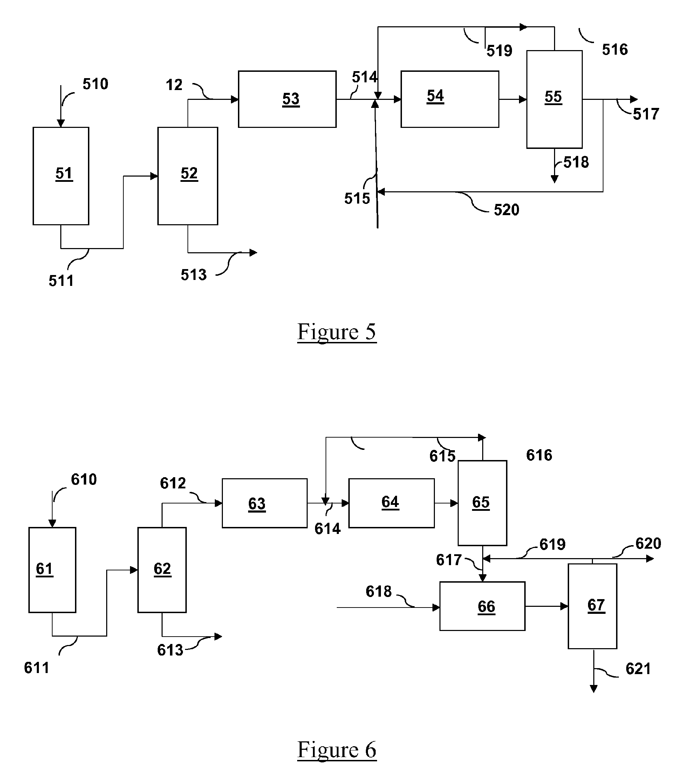

Figure 5 shows an alkylation process scheme using a purification section and a recycle of unreacted olefin and aromatic streams.

The untreated refinery stream 510 is fed to a Selective Hydrogenation Unit (SHU) 51. The dedienized refinery stream 511 thus obtained is fed to a first splitter 52 wherein it is separated in LCCS 512 (FBP = 60°C to 100°C) and M/HCCS 513 (Boiling point cut ranges from 60-170°C to 100-170°C).

LCCS 512 is then fed to a purification unit 53 for removal of nitrogen by adsorption on sorbents.

The purified LCCS stream 514 obtained is fed to an alkilation unit 54, the obtained products being separated in a further splitter 55 into a gasoline 516, aromatics 517 and middle distillates 518.

The part of the gasoline 516, consisting of unreacted olefins, may optionally be recycled back into the alkylation unit 54 via the line 519 as well as the part of unreacted aromatic 517 via line 520.

An aromatic containing stream (e.g. reformate), may also be added to the feed of alkylation unit 54 via line 515.

Figure 6 shows an oligomerization and alkylation process scheme using a purification section and optionally a recycle of unreacted olefin and aromatic streams.

The untreated refinery stream 610 is fed to a Selective Hydrogenation Unit, SHU 61. The dedienized refinery stream 611 thus obtained is separated in a first splitter 62 into a LCCS 612 (FBP = 60°C to 100°C) and a M/HCCS 613 (Boiling point cut ranges from 60-170°C to 100-170°C).

LCCS 612 is then purified in a purification unit 63 for removal of nitrogen by adsorption on sorbents.

The purified stream 614 is fed to an oligomerization unit 64.

The effluents issued from the oligomerization unit 64 are separated in another splitter 65 into a stream 616 of oligomers and unreacted olefins, a part of which may optionally be recycle upstream of oligomerization unit 64 via line 615, and into a stream of (light) middle distillates 617.

Middle distillates 617 are fed to an alkylation unit 66 which is also fed with a (light) gasoline cut 618. Effluents issued from the alkylation unit 66 are separated in a third splitter 67 into a (heavier) gasoline cut 620, a part of which may be recycled back upstream of alkylation unit 66 via line 619, and into a (heavier) middle distillate 621.

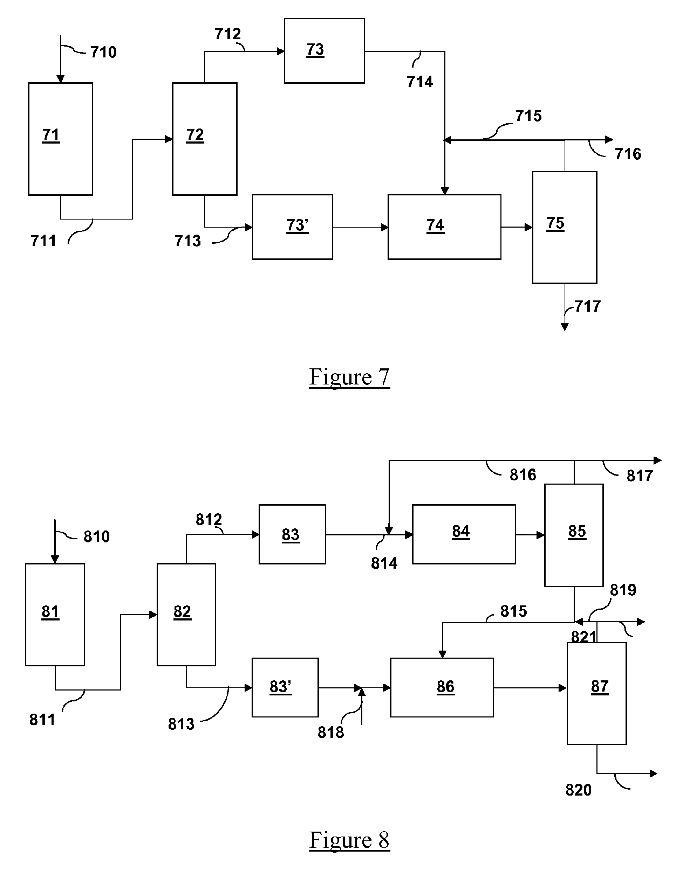

Figure 7 shows a one pot oligomerization and alkylation process scheme using a purification section and optionally a recycle of unreacted olefin stream.

The untreated refinery stream 710 is fed to a Selective Hydrogenation Unit, SHU 71. The dedienized refinery stream 711 thus obtained is separated in a first splitter 72 into a LCCS 712 (FBP = 60°C to 100°C) and a M/HCCS 713 (Boiling point cut ranges from 60-170°C to 100-170°C).

LCCS 712 is then purified in a dedicated purification unit 73 and M/HCCS 713 is purified in another dedicated purification unit 73', for removal of nitrogen by adsorption on sorbents.

The purified streams 714 and 718 issued respectively from purification units 73 and 73', are fed to a combined oligo-alkylation unit 74.

The effluents issued from the oligo-alkylation unit 74 are separated in a further splitter 75 into a stream 716 of middle distillates, a part of which may optionally be recycled upstream of oligo-alkylation unit 74 via line 715, and into a gasoline cut 717.

Figure 8 shows an oligomerization and alkylation process scheme of a full olefin containing refinery stream, this process using a purification section and optionally a recycle of unreacted olefin and aromatic streams.

The untreated refinery stream 810 is fed to a Selective Hydrogenation Unit, SHU 81. The dedienized refinery stream 811 thus obtained is separated in a first splitter 82 into a LCCS 812 (FBP = 60°C to 100°C) and a M/HCCS 813 (Boiling point cut ranges from 60-170°C to 100-170°C).

LCCS 812 is then purified in a purification unit 83 on sorbents and M/HCCS 813 is purified in a different purification unit 83', for removal of nitrogen by adsorption on sorbents.

The purified LCCS stream 814 is fed to an oligomerization unit 84. The effluents issued from the oligomerization unit 84 are separated in another splitter 85 into a stream 817 of (light) gasoline cut and a stream 815 of oligomers. The part of unreacted olefins 816 of the stream 817 may be recycled upstream of oligomerization unit 84.

The purified M/HCCS 813 is fed to an alkylation unit 86, preferably with an aromatic containing stream 818. The stream 815 of oligomers issued from splitter 85 is also fed to the alkylation unit 86 The effluents issued from the alkylation unit 86 are separated in a further splitter 87 into a stream 820 of middle distillates and a stream 821 of a (heavier) gasoline cut. The part of unreacted material 819 of the stream 821 may be recycled upstream of alkylation unit 86.

## Examples

In the following examples, the gasoline cut feedstock is a LCCS cut (Light Catalytic Cracked Stream) corresponding to the low boiling point fraction of a LCN (Light Cracked Naphtha) treated on a Prime-G 1st stage unit (Prime-G is a naphtha selective hydrogenation technology marketed by Axens, which hydrogenates most reactive alkenes, mainly di-olefines, in particular conjugated dienes (e.g. buta-1,3-diene into but-1-ene) and eventually isomerizes n-olefines (end-chain double bond, e.g. n-hex-1-ene) into sec-olefines (internalized double bond, e.g. n-hex-2-ene), so as to get rid of the di-olefins (by selective hydrogenation) and of the low molecular weight sulfur containing molecules by conversion into heavier ones.

Characteristics of the gasoline cut are reported below in table 1.

Table 1 - Characteristics of the LCCS cut used in the examples

|  | Unit | Value |
|---|---|---|
| Density at 15°C | g/mL | 0.6518 |
| Sulfur | ppm wt | 24 |
| Total nitrogen | ppm | 12.6 |
| Diene value UOP 326 | g iodine/ 100g | 0.11 |
| Bromine Number ASTM D1159 | g bromine/100g | 140.8 |
| Reid Vapor Pressure ASTM D5191 | kPa | 125.5 |
| **Sulfur speciation** |  |  |
| Methyl-ethyl-sulfide | ppm wt | 2 |
| Thiophene | ppm wt | 22 |
| **ASTM D86** |  |  |
| T°C at IBP | °C | 27.6 |
| T°C at 5% vol | °C | 31 |
| T°C at 50% vol | °C | 38 |
| T°C at 95% vol | °C | 55 |
| T°C at FBP | °C | 63.6 |

(continued)

| Chemical species | | |
|---|---|---|
| C4 | % wt | 6.80 |
| C5 | % wt | 45.44 |
| C6 | % wt | 10.65 |
| Total olefins | % wt | 62.89 |
| iC5 | % wt | 21.35 |

The olefinic conversion is expressed in %wt as:

$$100 \text{ x (olefin IN} - \text{olefin OUT)/olefin IN}$$

100mL of ZSM-5 based catalyst diluted with 100mL of inert material (SiC 0.21mm) have been loaded in a fixed bed tubular reactor of 18mm inner diameter. Before testing, catalyst has been activated at 400°C (60°C/h) under 160NL/h Nitrogen during 2 hours. Temperature has then been decreased down to 40°C before starting the testing program.

Example 1 (comparative example): oligomerization without purification section

The LCCS cut is directly oligomerized on a ZSM-5 based catalyst (80%wt alumina - 20%wt ZSM-5) in the following operating conditions: at 55barg, with a LHSV (liquid hour space velocity) of $1h^{-1}$, once through, temperature has been increased from 180°C, to 220°C, up to 250°C.

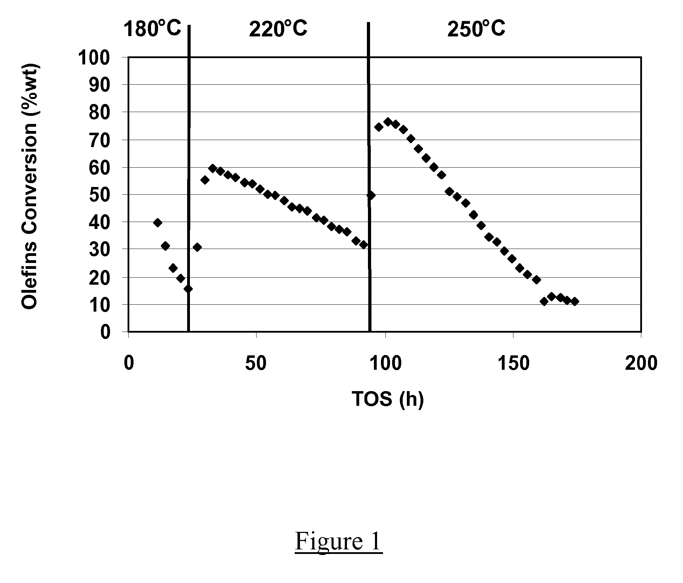

Olefin conversion (%wt) data as a function of TOS when no purification section is used is shown at figure 1.

Though the contaminants level is low (24ppmwt S, 12ppmwt N), the deactivation of the catalyst is significantly pronounced as represented in the figure 1: for instance at 220°C, a loss of 28%wt olefin conversion is observed within 59 hours. Table 2 below gathers the product characteristics obtained for samples withdrawn at different times of stream (TOS).

Table 2 - Product distribution and density of the effluents recovered at different TOS when no purification section is used

| | 180°C | 220°C | | | 250°C | |
|---|---|---|---|---|---|---|
| TOS (h) | 16.3 | 31 | 51 | 75 | 116 | 147 |
| IBP-165 | 96.2 | 83.2 | 85.5 | 90.9 | 81 | 94.2 |
| 165-245 | 2.2 | 12.3 | 10.3 | 5.8 | 14.6 | 3.7 |
| 245-350 | 0 | 0 | 0 | 0 | 2.7 | 0 |
| 350-FBP | 0 | 0 | 0 | 0 | 0 | 0 |
| FBP (°C) | 194 | 266 | 263 | 196 | 272 | 194 |
| Density at 15°C (g/mL) | 0.6642 | 0.6899 | 0.6847 | 0.675 | 0.6924 | 0.6652 |

This example clearly stresses the need for a process sequence in which catalyst lifetime can be improved.

Example 2: oligomerization with purification section

The LCCS cut is first purified on a set of two molecular sieves : 3A followed by 13X, before being oligomerized on the ZSM-5 based catalyst (80%wt alumina - 20%wt ZSM-5) used for example 1.
The following operating conditions were chosen : 55barg, a LHSV (liquid hour space velocity) of $1h^{-1}$, once through, and temperature has been increased from 180°C, to 220°C, up to 250°C.

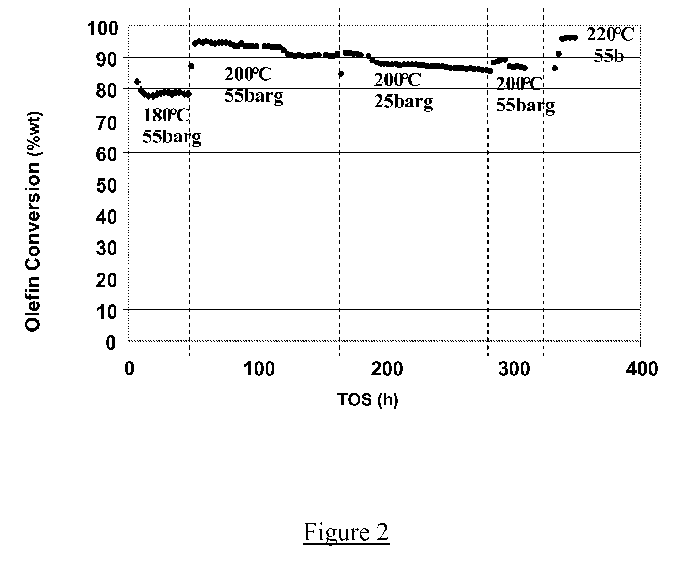

As observed on figure 2, the catalyst deactivation has been largely limited thanks to the use of a purification section.

The product distribution as well as the density of the effluent recovered is reported in table 3 below. The following abbreviations are used:

WABT = Weight Average Boiling Point.
Barg = bar gauge, i.e. absolute pressure minus atmospheric pressure. 1barg = 2bar absolute (or 2bara) when referred to a complete vacuum, and when atmospheric pressure is 1 bar.

Analyses of the LCCS purified on molecular sieves have been achieved and reveal that:

- The breakthrough of sulfur compounds (mainly thiophenic) is quickly reached (after only 2 days of run, the same sulfur content is obtained at the outlet of the molecular sieves)
- Nitrogen containing species are successfully retained on the purification section (the N content is found below 0.5ppmwt).

The comparison of the results obtained in presence and in absence of a purification section (figures 1&2) clearly underlines the beneficial effect of the use of a purification section prior to acid catalyzed reactions (such as oligomerization, alkylation ...). The larger impact of small nitrogen containing molecules compared to aromatic sulfur containing molecules (e.g. thiophene) on catalyst deactivation is also clearly stressed here.

Boiling points are given at ambient pressure, unless otherwise specified.

Table 3 - Product distribution (%) and density of the effluents recovered at different TOS when a purification section is used.

| Pressure in operation | 55 barg | | | | | | |
|---|---|---|---|---|---|---|---|
| TOS(h) | 16 | 31.5 | 55.5 | 79.5 | 103.5 | 127.5 | 151.5 |
| WABT (°C) | 182.2 | 182.2 | 202.9 | 202.8 | 202.7 | 198.5 | 199.6 |
| | | | | | | | |
| IBP-165 | 76.7 | 76.6 | 65.8 | 66.3 | 66.5 | 69.3 | 69.5 |
| 165-245 | 17 | 17 | 21.3 | 21.3 | 21.1 | 20.5 | 20.5 |
| 245-350 | 3.6 | 3.1 | 10.4 | 10 | 10 | 8.9 | 8.7 |
| 350-FBP | 0 | 0 | 0 | 0 | 0 | 0 | 0 |
| FBP (°C) | 271 | 269 | 329 | 326 | 326 | 320 | 318 |
| Density at 15°C (g/mL) | 0.7036 | 0.7033 | 0.716 | 0.7156 | 0.7153 | 0.713 | 0.7128 |

| Pressure in operation | 25 barg | | | | 55 barg | |
|---|---|---|---|---|---|---|
| TOS(h) | 175.5 | 223.5 | 247.5 | 271.5 | 295.5 | 343.5 |
| WABT (°C) | 203.5 | 200.2 | 200.2 | 200.1 | 199.4 | 223.1 |
| | | | | | | |
| IBP-165 | 67.8 | 70.7 | 71.5 | 71.7 | 71.5 | 60 |
| 165-245 | 20.7 | 20.1 | 19.5 | 19.4 | 20.2 | 23 |
| 245-350 | 9.6 | 8.2 | 7.6 | 7.2 | 6.8 | 14.2 |
| 350-FBP | 0 | 0 | 0 | 0 | 0 | 1 |
| FBP (°C) | 323 | 319 | 317 | 316 | 316 | 360 |

(continued)

| Pressure in operation | 25 barg | | | | 55 barg | |
|---|---|---|---|---|---|---|
| Density at 15°C (g/mL) | 0.7141 | 0.7106 | 0.7099 | 0.7093 | 0.7104 | 0.7184 |

**Claims**

1. A method for the treatment of an olefin containing hydrocarbon feedstock, wherein said feedstock successively undergoes (i) selective hydrogenation, (ii) treatment on adsorbent to obtain at least one nitrogen depleted fraction, (iv) at least one middle distillate generation via (iv-a) oligomerization of said nitrogen depleted fraction and/or (iv-b) alkylation of said nitrogen depleted fraction.

2. A method according to claim 1, wherein (v) at least one unreacted material is separated from said at least one middle distillate.

3. A method according to claim 2, wherein said at least one unreacted material is recycled at step (iv) for at least one middle distillate generation.

4. A method according to one any of the preceding claims, wherein selectively hydrogenated feedstock is splitted into at least two of LCCS, MCCS, HCCS and fuel gas prior to treatment of at least one of the foregoing on adsorbent.

5. A method according to one any of the preceding claims, wherein alkylation of said at least one nitrogen depleted fraction is performed in presence of an aromatic containing hydrocarbon feedstock.

6. A method according to one any of claims 1-5, wherein said feedstock successively undergoes (i) selective hydrogenation, (ii) splitting into a light cut (LCCS) and a heavier cut (HCCS or mixed MCCS/HCCS), (iii) LCCS treatment on adsorbent to obtain a nitrogen depleted LCCS, (iv) a first middle distillate generation via (iv-a) oligomerization of said nitrogen depleted LCCS and/or (iv-b) alkylation of said nitrogen depleted LCCS.

7. A method according to claim 6, wherein (vi) said first middle distillate is further alkylated with a gasoline cut to produce a second middle distillate.

8. A method according to claim 7, wherein (vii) a second unreacted material is separated from said second middle distillate.

9. A method according to claim 8, wherein said second unreacted material is recycled at step (vii) for said second middle distillate generation.

10. A method for the treatment of an olefin containing hydrocarbon feedstock according to one any of claims 1-5, wherein said feedstock successively undergoes (i) selective hydrogenation, (ii) splitting into a light cut (LCCS) and a heavier cut (HCCS or mixed MCCS/HCCS), (iii) said LCCS and said HCCS or mixed MCCS/HCCS separate treatment on adsorbent so as to remove nitrogen compounds, to obtain nitrogen depleted LCCS and nitrogen depleted HCCS or mixed MCCS/HCCS, (iv) a third middle distillate generation via (iv-a) oligomerization and/or (iv-b) alkylation of said nitrogen depleted LCCS combined with said nitrogen depleted HCCS or mixed MCCS/HCCS.

11. A method according to claim 10, wherein a gasoline cut is separated from said third middle distillate.

12. A method for the treatment of an olefin containing hydrocarbon feedstock according to one any of claims 1-5, wherein said feedstock successively undergoes (i) selective hydrogenation, (ii) splitting into a light cut (LCCS) and a heavier cut (HCCS or mixed MCCS/HCCS), (iii) said LCCS and said HCCS or mixed MCCS/HCCS separate treatment on adsorbent so as to remove nitrogen compounds, to obtain nitrogen depleted LCCS and nitrogen depleted HCCS or mixed MCCS/HCCS, (iv-a) a fourth middle distillate generation via oligomerization of said nitrogen depleted LCCS, (iv-b) a fifth middle distillate generation via alkylation of said nitrogen depleted HCCS or mixed MCCS/HCCS.

13. A method according to claim 12, wherein unreacted olefins are separated from said fourth middle distillate.

14. A method according to claim 13, wherein unreacted olefins are recycled at step (iv-a) for fourth middle distillate generation.

15. A method according to claim 12, wherein fourth middle distillate is alkylated with nitrogen depleted HCCS or mixed MCCS/HCCS and an aromatic containing stream at step (iv-b) to produce said fifth middle distillate.

16. A method according to claim 15, wherein unreacted material is separated from said fifth middle distillate.

17. A method according to claim 16, wherein unreacted material is recycled at step (iv-b).

18. A method according to one any of the preceding claims, wherein said adsorbent comprises one or more of molecular sieves, acidic ion-exchange resins, acid-treated clays, activated aluminas, spent FCC catalysts, MOF (Metal-Organic Framework), ASA, NiMo, and catalysts guard beds.

19. A method according to claim 18, wherein the adsorbent is selected among, or is a combination of one or more of 13X molecular sieve, ASA, NiMo, and MOF.

20. A method according to one any of the preceding claims, wherein said adsorbent is loaded into a purification section located in a guard bed capacity.

21. A method according to claim 20, wherein the guard bed capacity is operated on a swing cycle with two or three beds, and wherein, respectively, one or two beds in series, are being used on stream for contaminant removal, while the remaining one is on regeneration.

22. A method according to one any of the preceding claims, wherein oligomerization and/or alkylation catalyst is selected among amorphous or crystalline alumosilicates or silicaalumophosphates in H-form, which are optionally containing alkali, alkali-earth, transition or rare-earth element, said catalyst being selected from MFI, MEL (Si/Al >25), ASA (amorphous silica-alumina), MSA (mesoporous silica-alumina), FER, MTT, MWW, TON, EUO, ZSM-48, MFS, MTW, MAZ, SAPO-11, SAPO-5, FAU, LTL, BETA, MOR, SAPO-40, SAPO-37, SAPO-41, MCM-41, MCM-48 and a family of microporous materials consisting of silicon, aluminum, oxygen and optionally boron, $Al_2O_3$, and mixtures thereof.

23. A method according to claim 22, wherein said catalyst is selected among ZSM-5, silicalite-1, boralite C, TS-1, ZSM-11, silicalite-2, boralite D, TS-2, SSZ-46, Ferrierite, FU-9, ZSM-35, ZSM-23, MCM-22, PSH-3, ITQ-1, MCM-49, ZSM-22, Theta-1, NU-10, ZSM-50, EU-1, ZSM-48, ZSM-57, MTW, MAZ, SAPO-11, SAPO-5, USY, LTL, BETA, MOR, SAPO-40, SAPO-37, SAPO-41, MCM-41, MCM-48, $Al_2O_3$, and mixtures thereof.

24. A method according to claim 22, wherein said catalyst is selected among amorphous alumosilicates or silicaalumophosphates in H-form, optionally modified by addition of halogen, selected from MSA (mesoporous silica-alumina).

25. A method according to claim 24, wherein the halogen is fluorine.

26. A method according to one any of claims 22 to 24, wherein the catalyst is subjected to a treatment in an alkaline medium before use.

27. A method according to one any of the preceding claims, wherein the amount of alkali, alkali-earth, transition or rare-earth element is in the range 0.05-10wt%.

28. A method according to claim 27, wherein the amount of alkali, alkali-earth, transition or rare-earth element is in the range 0.1-5wt%.

29. A method according to claim 28, wherein the amount of alkali, alkali-earth, transition or rare-earth element is in the range 0.2-3wt%.

30. A method according to claim 22 to 30, wherein the element is selected among Ba, Ca, Ce, K, La, Mg, Na, Sr, and mixtures thereof.

31. A method according to claim 22 to 30, wherein the catalyst is additionally doped with further metal in at least 0.1wt%.

**32.** A method according to claim 31, wherein the metal is selected among Zn, Mn, Co, Ni, Ga, Fe, Ti, Zr, Ge, Sn, Cr, and mixtures thereof.

**33.** A method according to claims 22 to 32, wherein the catalyst is a sole catalyst or a blend of catalysts according to claims 23 to 33.

**34.** A method according to claim 33, wherein the sole catalyst or blend of catalysts is combined with a binder or a matrix.

**35.** A method according to one any of the preceding claims, wherein the feedstock issues from FCC, coker, flexi-coker, visbreaker, steam cracker, hydrocracker.

**36.** A method according to claim 35, wherein the feedstock issues from DHC or MHC hydrocracker.

**37.** A method according to claim 35, wherein the feedstock issues from FCC or coker.

**38.** A method according to one any of claims 35 to 37, wherein the final boiling point of the feedstock is below 200°C.

**39.** A method according to claim 38, wherein the final boiling point of the feedstock is below 165°C.

**40.** A method according to one any of claims 35 to 39, wherein the initial boiling point of the feedstock is above -50°C.

**41.** A method according to claim 40, wherein the initial boiling point of the feedstock is above 0°C.

**42.** A method according to claim 41, wherein the initial boiling point is above +25°C.

Figure 1

Figure 2

Figure 3

Figure 4

Figure 5

Figure 6

Figure 7

Figure 8

Europäisches
Patentamt

European
Patent Office

Office européen
des brevets

# EUROPEAN SEARCH REPORT

Application Number

EP 10 30 5759

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | FR 2 879 620 A1 (INST FRANCAIS DU PETROLE [FR]) 23 June 2006 (2006-06-23)<br>* example 1 *<br>----- | 1-42 | INV.<br>C10G25/05<br>C10G29/20<br>C10G45/32 |
| X | US 2008/302704 A1 (KECKLER KENNETH PAUL [US] ET AL) 11 December 2008 (2008-12-11)<br>* paragraph [0026] *<br>* the whole document *<br>----- | 1-42 | C10G50/00<br>C10G69/12<br>C07C2/00 |
| X | US 2005/137442 A1 (GAJDA GREGORY J [US] ET AL) 23 June 2005 (2005-06-23)<br>* paragraph [0005] *<br>* the whole document *<br>----- | 1-42 | |
| X | US 2008/312484 A1 (GODSMARK JOHN S [BE] ET AL) 18 December 2008 (2008-12-18)<br>* paragraph [0048] *<br>* the whole document *<br>----- | 1-42 | |
| X | US 2006/270886 A1 (BROWN STEPHEN H [BE] ET AL BROWN STEPHEN HAROLD [BE] ET AL) 30 November 2006 (2006-11-30)<br>* paragraph [0034] *<br>* paragraph [0060] - paragraph [0061] *<br>----- | 1-42 | TECHNICAL FIELDS SEARCHED (IPC)<br><br>C10G<br>C07C<br>B01J |

The present search report has been drawn up for all claims

| Place of search | Date of completion of the search | Examiner |
|---|---|---|
| The Hague | 14 December 2010 | Bernet, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 30 5759

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2010

| Patent document cited in search report | | Publication date | Patent family member(s) | Publication date |
|---|---|---|---|---|
| FR 2879620 | A1 | 23-06-2006 | CN 101084291 A | 05-12-2007 |
| | | | DE 112005003177 T5 | 24-01-2008 |
| | | | WO 2006067305 A1 | 29-06-2006 |
| | | | GB 2437203 A | 17-10-2007 |
| | | | JP 2008524421 T | 10-07-2008 |
| | | | US 2010036182 A1 | 11-02-2010 |
| US 2008302704 | A1 | 11-12-2008 | NONE | |
| US 2005137442 | A1 | 23-06-2005 | NONE | |
| US 2008312484 | A1 | 18-12-2008 | CN 101198568 A | 11-06-2008 |
| | | | CN 101198569 A | 11-06-2008 |
| | | | EP 1912923 A1 | 23-04-2008 |
| | | | WO 2007006398 A1 | 18-01-2007 |
| | | | WO 2006133967 A1 | 21-12-2006 |
| US 2006270886 | A1 | 30-11-2006 | BR PI0610054 A2 | 25-05-2010 |
| | | | CA 2609804 A1 | 07-12-2006 |
| | | | CN 101208413 A | 25-06-2008 |
| | | | EP 1888715 A2 | 20-02-2008 |
| | | | JP 2008542469 T | 27-11-2008 |
| | | | KR 20080007262 A | 17-01-2008 |
| | | | US 2010282641 A1 | 11-11-2010 |
| | | | WO 2006130248 A2 | 07-12-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080039669 A
- US 20080312484 A
- US 20080257783 A
- WO 2006091986 A
- US 4150062 A
- US 4227992 A
- US 4482772 A
- US 4506106 A
- US 4543435 A
- US 20040254413 A

- WO 2006076942 A
- US 5063187 A
- US 6884916 B
- US 7253330 B
- US 7186874 B
- WO 2007104385 A
- WO 2006094010 A
- US 20050137442 A
- EP 1433835 A
- WO 2006067305 A